# EUROPEAN PATENT SPECIFICATION

(11) **EP 3 065 752 B1**
(45) Date of publication and mention of the grant of the patent: **12.06.2019**
(21) Application number: 14857614.3
(22) Date of filing: 28.04.2014
(51) Int. Cl.: A61K 36/899, A61P 19/00

(54) **NOVEL USE OF A BAMBOO FOR PROMOTING BONE GROWTH**
NEUARTIGE VERWENDUNG VON BAMBUS ZUR FÖRDERUNG DES KNOCHENWACHSTUMS
NOUVELLE UTILISATION D'UN BAMBOU POUR STIMULER LA CROISSANCE OSSEUSE

(30) Priority: 04.11.2013 KR 20130133194
(43) Date of publication of application: 14.09.2016
(73) Proprietor: Novatec Co., Ltd., Seongnam-si, Gyeonggi-do 13201 (KR); Kim, Hohyun, Anyang-si, Gyeonggi-do 431-050 (KR); Kim, Sooyeon, Anyang-si, Gyeonggi-do 431-050 (KR)
(72) Inventor: KIM, Hohyun, 124-1804, 135, Gwanak-daero, Dongan-gu, Anyang-si (KR); KIM, Sooyeon, 124-1804, 135, Gwanak-daero, Dongan-gu, Anyang-si (KR); JEONG, Ji Hoon, 113-1802, 43, Wongol-ro, Uiwang-si (KR)
(74) Representative: Staeger & Sperling Partnerschaftsgesellschaft mbB
(86) International application number: PCT/KR2014/003727
(87) International publication number: WO 2015/064865

(56) References cited:
- JP-A- 2009 221 093
- KR-A- 20020 061 379
- KR-A- 20070 006 797
- KR-A- 20080 000 261
- KR-A- 20120 033 633
- US-A1- 2008 279 969
- HOQUE M ET AL: "Evaluation of alcoholic extract of bamboo (Bambusa arundinacea) buds on fracture healing in rabbit", INDIAN JOURNAL OF ANIMAL SCIENCES, vol. 74, no. 9, September 2004 (2004-09), pages 915-917, XP009193762, ISSN: 0367-8318
- KIM HYUNG-RYONG ET AL: "Effect of ginseng mixture on osteoporosis in ovariectomized rats", IMMUNOPHARMACOLOGY AND IMMUNOTOXICOLOGY, vol. 30, no. 2, 2008, pages 333-345, XP009193746, ISSN: 0892-3973
- CHUNG YOON HEE ET AL: "Effects of Aqueous Extract of Phyllostachyos Caulis in Taeniam on Longitudinal Bone Growth in Adolescent Rats", PLANTA MEDICA, vol. 82, no. 4, March 2016 (2016-03), pages 330-336, XP055355538,

## Description

### [Technical Field]

This application claims priority from and the benefit of Korean Patent Application No. 10-2013-0133194 filed on November 4, 2013.

This invention is directed to a novel non-therapeutic use of a bamboo composition for promoting the growth of bone length according to claim 1.

### [Background Art]

As the body somatotype has recently been westernized and the average height has grown, more attentions are being paid to height growth factors. When defined in a broader sense, growth does not only mean the increase of height, but also the enlargement of each body organ and the enhancement of its functions. On the other hand, in a narrower sense, it can be defined as height increase. Such a height increase occurs through a skeletal metabolism including the synthesis of cartilaginous tissues, the longitudinal growth of bones and the comprehensive proliferation of skeletal tissues which are mediated by nutrition and growth hormones and so on. The skeletal tissue is a specially-differentiated connective tissue which is composed of several different types of cells such as mesenchymal cells, cartilage cells, osteoblasts, osteoclasts and marrow cells. The quantity of osteolysis by osteoclasts and osteogenesis by osteoblasts is kept well in balance. During the growth period when bone formation by osteoblasts reaches its maximum, the quantity of osteogenesis exceeds significantly that of osteolysis, resulting in bone growth.

A growth plate is a group of cartilage cells which is a horizontally patterned structure and located between the metaphysis and the epiphysis at both ends of the long bone. Cartilage inside the growth plate goes through the conversion of cartilage into bone, i.e. the process of endochondral ossification which is comprised of the proliferation and hypertrophy of chondrocytes, the secretion of extracellular matrix, the invasion of blood vessels and osteo-progenitor cells and the resulting ossification. Following such a process, a new bone continues to form at the end of the bone body adjoining the growth plate, leading to the increase in the length of the long bone. While the growth plate is affected by such hormones as growth hormone, IGF (insulinlike growth factor), thyroid hormone, sex hormone and glucocorticoid as well as various cytokines, it eventually ossifies after adolescence and gets fused with adjacent bones (See Shin Choong Ho, Hormonal Regulation of Growth Plate, Korean Journal of Pediatrics, Vol.11,117-122,2006).

Until now, such methods have been introduced to promote bone growth as the administration of growth hormones, Ilizarov operation and the intake of nutritional supplements. Particularly, as people have recently had more interests in height growth, growth hormone treatment, which used to be applied mainly to pathological poor growth, is currently being applied to children and teenagers of normal height in their growth period. The above-mentioned growth hormone treatment indeed shows an excellent effect on those who lack in growth hormone. On the contrary, when applied to those who have the normal level of growth hormone, it may cause most of them various side effects such as acromegaly, the development of anti-growth hormone antibodies, systematic allergic reaction and hypothyroidism. Further, while it requires a long-term administration and high expenses, other problems such as the risk of cancer and the disturbance of relevant growth factors have been found. Furthermore, since Ilizarov surgery is an operational procedure in which a leg bone is cut and stretched, it has been found inappropriate for general population considering that it is too painful and expensive. Lastly, most nutritional supplements for growth promotion have yet to be scientifically verified for their efficacy. Therefore, there is a need for developing a safe and scientifically-verified food substance for promoting growth.

Further prior art in this technical field is disclosed in document KR 2002 0061379 A.

### [Disclosure]

### [Technical Problem]

In this respect, while the inventors of the present invention have been researching on the development of a food substance which is capable of promoting bone growth effectively and without side effects, they have found the effectiveness of bamboo in enhancing the growth plates and increasing the length of the long bones in the bamboo-treated test animals, leading to the completion of the present invention.

Accordingly, the object of the present invention is to provide a non-therapeutic composition for promoting bone growth which comprises a bamboo as an effective ingredient.

### [Technical Solution]

To achieve the above-mentioned object, the present invention provides a non-therapeutic use of a composition for promoting bone growth according to claim 1.

Bamboo is a general name of perennial plants belonging to the Sub-Family of *Bambusoideae*, the Family of *Gramineae,* and the Order of *Graminales.* It is known that there are about 280 species of bamboo under the Family of *Gramineae* around the world. In Korea, about 70 species of bamboo are known to grow in the wild or be cultivated.

There are 11 representative species of bamboo: *Phyllostachys nigra varhenosis, Phyllostachys bambusoides Sieb. et Zucc., Phyllostachys pubescens, Phyllostachys nigra Munro, Phyllostachys nigra var. henonis f*. *punctata Nakai, Sasa borealis var. gracilis, Arundinaria simonii, Sasa chiisanensis, Sasa borealis* Makino, and *Pseudosasa japonica.* Among the above representative species of bamboo, major cultivation species are *Phyllostachys nigra var. henosis, Phyllostachys bambusoides Sieb. et Zucc.* and *Phyllostachys pubescens.*

Bamboo as used in the present invention includes, but is not limited to, any known species of bamboo. Preferably, it is at least one selected from the group consisting of *Phyllostachys nigra var. henosis, Phyllostachys bambusoides Sieb. et Zucc., Bambusa tuldoides and Sinocalamus beechenyanus var. pubesceus.* More preferably,it is *Phyllostachys nigra var. henosis* or *Phyllostachys bambusoides Sieb. et Zucc.*

While any part of bamboo can be used for the purpose of the present disclosure, it may be preferably at least one selected from the group consisting of leaves, resin, fresh sprouts, roots and the middle parts of branches whose outer layers are removed. According to the invention it is the middle parts of bamboo branches whose outer layers are removed. The middle parts of bamboo branches whose outer layers are removed are called Jukye ( 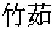 , *Bambusae Caulis In Taeniam*), Cheong Jukye (*Phyllostachys nigra var henonis*), Jukpi(bamboo sheath), or Damjuk Piye. Each of the above terms is used interchangeably to refer to the said middle parts of bamboo branches throughout the detailed description of the present application.

The bamboo according to the present disclosure can be used in its natural or processed form designed for the administration to animals.

The bamboo according to the present disclosure may be, but not limited to, in the form of powder, suspension and extract, while it is preferably a bamboo extract.

The bamboo extract can be prepared by known extraction methods for natural substances, preferably by using at least one solvent selected from the group consisting of water, a C1-C6 organic solvent and subcritical or supercritical fluid. The C1-C6 organic solvent can be selected from the group consisting of C1 to C6 alcohol, acetone, ether, benzene, chloroform, ethyl acetate, methylene chloride, hexane, cyclohexane, diethyl ether and petroleum ether. Preferably, the bamboo extract can be prepared by using water or C1 to C6 alcohol. The bamboo extract can be obtained by utilizing a pre-treatment procedure (for example, pulverization with a grinder) in order to improve the extraction efficiency.

While the extraction temperature for the preparation of the bamboo extract according to the present invention is not particularly limited, it may vary between 0°C and 150°C, preferably between 80°C and 120°C. Further, while the extraction time according to the present invention is not particularly limited in relation with the extraction temperature, it may vary from an 1 hour to 10 days, preferably from 30 minutes to 6 hours, more preferably from an 1 hour to 3 hours.

The bamboo extract according to the present invention can be prepared by known extraction methods for natural substances. For instance, it can be prepared by macerating extraction, hot water extraction, ultrasonic wave extraction, reflux condensing extraction or heating extraction, preferably hot water extraction or reflux condensing extraction. The extraction frequency may vary from once to 10 times, preferably from 2 to 7 times.

While the bamboo extract of the present invention can be filtered and used in a liquid form, it can be preferably used in a solid form following a drying process such as spray drying or freeze-drying. More preferably, the bamboo extract may be mixed with dextrin prior to spray drying or freeze-drying.

According to the present invention the extract of *Bambusae caulis In Taeniam* is used. The extract of *Bambusae caulis In Taeniam* is not limited to, but preferably a liquid extracted from the inner layers of *Phyllostachys nigra var. henosis, Phyllostachys bambusoides Sieb. et Zucc., Bambusa tuldoides or Sinocalamus beechenyanus var*. *pubesceus.* In addition, the extract of *Bambusae caulis In Taeniam* is not limited to, but most preferably one prepared by using hot water extraction method with water as a solvent.

While the ratio of water to bamboo used during hot water extraction is not particularity limited, it may be 3 to 20: 1 (water:bamboo, weight basis), preferably 8 to 15: 1 in order to improve the efficiency of extraction.

In one example, the bamboo extract according to the present invention was prepared by grinding bamboo into the size of 20∼40 mesh, followed by the addition of drinking water to the obtained bamboo powder at the weight ratio of 1 to 10 (bamboo powder:water) and subsequently extracted by hot water extraction at 100°C for 2 hours. After the hot water extract was left still for 1 hour, a supernatant except for 10% of the extract sediment containing impurities was collected. Subsequently, the supernatant was freeze-dried to obtain the bamboo extract according to the present invention.

The term "bone growth" in the present invention means the increase in the size, thickness, density, length and function of the bone tissues, preferably the increase in the length of the bone.

The composition according to the present invention comprises bamboo as an effective ingredient and is effective for increasing bone length and promoting the proliferation of cartilaginous tissues in the growth plates.

The above said effectiveness of the composition according to the present invention is well demonstrated in the following examples of the detailed description.

In one example, after rats were administered with the bamboo extract of *Bambusae caulis In Taeniam* for 10 days, the change in the length of tibia was observed. It was found that the length of tibia in the bamboo extract-treated group increased similarly to that of the growth hormone-treated group.

In another example according to the present invention, after rats were administered with the bamboo extract of *Bambusae caulis In Taeniam* for 10 days, the change in the growth plate tissues was observed. It was also found that the bamboo extract-treated group showed a similar increase in the length of the proliferative zone and hypertrophic zone of the growth plates in comparison with the growth hormone-treated group.

In still another example of the present invention, after rats were administered with the bamboo extract of *Bambusae caulis In Taeniam* for 10 days, the change in the number of activated chondrocytes in cartilaginous tissues was observed by BrdU staining method. It was found that the ratio of BrdU positive cells increased, demonstrating that the proliferation of chondrocytes was promoted in the bamboo extract-treated group.

In still another example of the present invention, after rats were administered with the bamboo extract of *Bambusae caulis In Taeniam* for 10 days, the concentration of osteocalcin in the serum was measured. It was found that the concentration of osteocalcin in the serum increased, demonstrating that the bamboo extract of *Bambusae caulis In Taeniam* increased osteogenesis and the activity of osteoblast.

Therefore, the present disclosure provides a pharmaceutical composition effective for promoting bone growth, comprising a bamboo as an effective ingredient.

The pharmaceutical composition of the present disclosure may include bamboo according to the present invention solely or further contains one or more pharmaceutically acceptable carriers, excipients or diluents. The term "pharmaceutically acceptable" means a non-toxic substance/composition which, upon administering to human, is physiologically acceptable, does not hinder the function of an active ingredient and does not cause allergic or similar reactions, commonly such as gastrointestinal trouble and dizziness.

A pharmaceutically acceptable carrier may further include, for example, a carrier for oral or parenteral administration. A carrier for oral administration may contain lactose, starch, cellulose derivatives, magnesium stearate, stearic acid and the like. In addition, it may contain various drug delivery substances used for the oral administration of peptide agents. Furthermore, a carrier for parenteral administration may contain water, suitable oil, saline solution, aqueous glucose, glycol and the like, while additionally containing stabilizers and preservatives. A suitable stabilizer may include an antioxidant such as sodium hydrogen sulfite, sodium sulfite and ascorbic acid. A suitable preservative may include benzalkonium chloride, methyl- or propylparaben and chlorobutanol. Besides the above-mentioned ingredients, the pharmaceutical composition of the present disclosure may further comprise lubricants, humectants, sweeteners, flavoring agents, emulsifiers, suspending agents and the like. Other pharmaceutically acceptable carriers and agents as described in the following reference may be considered (See Remington's Pharmaceutical Sciences, 19th ed., Mack Publishing Company, Easton, PA, 1995).

The composition of the present invention may be administered to mammals including human beings in any manner. For instance, it may be administered either orally or parenterally. The parenteral administration may include, but not limited to, intravenous, intramuscular, intra-arterial, intramedullary, intradural, intracardiac, percutaneous, subcutaneous, intraperitoneal, intranasal, intestinal, topical, sublingual, or intra-rectal administration.

The pharmaceutical composition of the present disclosure may be formulated into an oral or parenteral formulation, depending on the above- mentioned administration routes.

In accordance with the known methods in the art, the oral formulation of the present invention may be prepared in the form of powder, granule, tablet, pill, sugar-coated tablet, capsule, liquid, gel, syrup, slurry, suspension and the like. For instance, the oral formulation of the present invention may be prepared in a tablet or sugar-coated tablet by mixing an active ingredient with a solid excipient, grinding the resulting mixture, adding a suitable adjuvant, and processing the resulting granule mixture.

A suitable excipient may include sugars such as lactose, dextrose, sucrose, sorbitol, mannitol, xylitol, erythritol and maltitol; starches such as corn starch, wheat starch, rice starch and potato starch; celluloses such as cellulose, methyl cellulose, sodium carboxymethylcellulose and hydroxypropyl methylcellulose; and fillings such as gelatin and polyvinyl pyrrolidone. Furthermore, cross-linked polyvinyl pyrrolidone, agar, alginic acid or sodium alginate may be added as a disintegrating agent- Still furthermore, the pharmaceutical composition of the present invention may additionally comprise anti-aggregating agents, lubricants, humectants, flavoring agents, emulsifiers and preservatives.

In accordance with the known methods in the art, the parenteral formulation of the present invention may be prepared in the form of injection, cream, lotion, externally-applying ointment, oil, moisturizer, gel, aerosol and nasal inhaler. The above-mentioned formulations are described in the following reference well known in the field of pharmaceutical chemistry (See Remington's Pharmaceutical Science, 19th ed., Mack Publishing Company, Easton, PA, 1995).

The composition of the present invention may be administered in a single dose, while it may be alternatively administered in a fractionated treatment protocol which utilizes a long-term, multiple dose regimen. The amount of an effective ingredient in the pharmaceutical composition according to the present disclosure may vary depending on the severity of disease. The preferable total amount of the pharmaceutical composition according to the present disclosure is 0.01 µg to 10,000 mg/day/kg (body weight), while 0.1 µg to 500 mg/day/kg (body weight) is the most preferable. However, the amount of the pharmaceutical composition is determined by considering various factors such as a patient's age, weight, health condition, gender, severity of disease, diet and excretion rate as well as formulation method, administration route and treatment frequency. Therefore, anyone having ordinary knowledge in the art may determine a suitable, pharmaceutically effective amount of the pharmaceutical composition according to the present invention by considering the above-mentioned various factors. The pharmaceutical composition of the present disclosure is not limited to a certain type of formulation, administration route and method as long as the effect of the present invention is maintained.

The present disclosure provides a food composition for promoting bone growth, comprising bamboo as an effective ingredient.

The food composition according to the present disclosure may include, but not limited to, any type of functional foods, nutritional supplements, health foods and food additives. The above-mentioned types of food composition may be prepared in accordance with the well-known methods in the art.

For example, bamboo according to the present invention may be consumed as a health food in the drinking form of tea, juice and drink, or in the ingestion form of granule, capsule and powder. In addition, bamboo according to the present invention may be prepared in the form of composition by combining with other substances or active ingredients which are known to be effective for promoting bone growth. For instance, in addition to bamboo or bamboo extract, the food composition according to the present disclosure may additionally contain a trace of minerals, vitamins, lipids, sugars and other ingredients known for their effectiveness in promoting growth. The above-mentioned minerals may include nutrients necessary during the growth period, such as calcium (Ca) and iron (Fe). The above-mentioned vitamins may include Vitamin C, E, B1, B2, B6 and the like. The above-mentioned lipids may include alkoxyglycerol, lecithin and the like. The above-mentioned sugars may include fructooligosaccharide and the like.

Furthermore, for the preparation of health foods, bamboo according to the present disclosure may be added to drinks (including alcoholic drinks); fruits and their processed products (e.g., canned fruit, bottled fruit, jam, marmalade and the like); fish, meat and their processed products (e.g., ham, sausage, corned beef and the like); breads and noodles (e.g., Udong, buckwheat noodle, ramen, spaghetti, macaroni and the like); fruit juices; various drinks; cookies; taffies; dairy products (e.g., butter, cheese and the like); edible vegetable oils; margarine; vegetable proteins; retort pouches; frozen foods; and various condiments (e.g. bean paste, soy sauce, sauce and the like).

In addition, bamboo according to the present disclosure may be prepared in the form of powder or concentrate in order to be used as a food additive.

The suitable amount of bamboo in the food composition according to the present invention is 0.01∼90 weight% (on the basis of the total weight of the food composition), while it is preferably 0.1∼50 weight%.

The present disclosure provides an animal feed composition for promoting bone growth, comprising bamboo as an effective ingredient.

The animal feed composition according to the present disclosure may be prepared in the form of fermented feed, assorted feed, pellet, silage and the like. The above-mentioned fermented feed may be prepared by comprising bamboo according to the present disclosure and additionally adding various microorganisms or enzymes for organic fermentation. The above-mentioned assorted feed may be prepared by mixing bamboo according to the present inv disclosure ention with several types of general feeds. The feed in the form of pellet may be prepared by formulating the above-mentioned fermented feed or assorted feed with a pelletizer. The silage may be prepared by mixing fresh, green forage with bamboo according to the present disclosure, followed by fermenting with lactic acid bacteria.

The present disclosure provides a method for promoting bone growth, the method comprising administering to a subject in need thereof an effective amount of a bamboo.

As used herein, the term "an effective amount" means an amount of a bamboo according to the present disclosure which is effective for promoting bone growth. As described above, the bamboo according to the present disclosure can be used in its natural or processed form designed for the administration to animals. Furthermore, the bamboo according to the present disclosure may be, but not limited to, in the form of powder, suspension and extract, while it is preferably a bamboo extract.

As described herein, the term "a subject" means an animal, preferably a mammal (especially, an animal including a human being), while it may include a cell, a tissue or an organ derived from an animal. Such a subject may include a patient for whom a treatment is needed.

### [Advantageous Effects]

Due to its effectiveness in promoting the growth of the growth plates and the long bones, the composition of the present invention comprising bamboo as an effective ingredient is effective for enhancing the growth and the formation of skeletal frame in infants, children and teenagers during their growth period. In addition, the composition of the present invention is effective for promoting height growth when used alone or in combination with growth hormone therapies.

### [Description of Drawings]

FIG. 1 illustrates the change in the body weight of rats in each group over the course of ten (10) days.
FIG. 2 shows the images of micro-CT scan on the tibia of rats in each group.
FIG. 3 shows the X-ray images on the tibia of rats in each group.
FIG. 4 illustrates the total length of the tibia in rats of each group.
FIG. 5 shows the location and the relative length of the proliferative zone and the hypertrophic zone within the growth plate in rats of each group.
   (Red arrow - the proliferative zone; Green arrow the hypertrophic zone; Control Saline-treated group; GH - Growth hormone-treated group;
20∼40 mesh. Drinking water was added to the obtained bamboo power at the ratio of 1:10 (bamboo powder:water), followed by hot water extraction at 100°C for 2 hours. After the hot water extract was left to stand for 1 hour, a supernatant except for 10% of the extract sediment containing impurities was collected. Subsequently, the supernatant was freeze-dried at -50°C for 3 hours to obtain the powder of the bamboo extract according to the present invention.

### <Experiment 1>

### Effect of the bamboo extract according to the present invention on the promotion of bone growth in animal models

### <1-1> Administration of the Bamboo Extract

3 weeks-old, male Sprague-Dawley rats were obtained and stabilized for 3 days. The feeding conditions included the temperature of 24±2°C, the light-dark cycle of 12 hours, and common solid feed without the inclusion of antibiotics. While the Control group was treated with saline solution, the bamboo (*Bambusae caulis in Taeniam)* extract, which was prepared in the powder form as described in the above example 1 and added with water, was orally administered once daily. The Positive Control group was treated subcutaneously with the injection of Eutropin (LG Life Sciences, South Korea) at 20 µg/kg once daily.

Regarding the administration method of the bamboo (*Bambusae caulis in Taeniam*) extract according to the present invention, rats in all the groups except for the Positive Control group were administered into their oral cavities, while all the rats were administered once daily.

The administered substances and dosages in each group are as follows:
- Control Group: normal saline solution 1 ml
- Positive Control Group: growth hormone 20 µg/kg
- Bamboo (*Bambusae caulis in Taeniam*)-treated Group: 200 mg/kg

After 10 days of feeding and administration of the above-mentioned substances, the rats were sacrificed with autopsy performed to find the following results.

### <1-2> Change in Body Weight of Experimental Animals

Rats of each group in Experiment <1-1> were tested for their body weight at pre- and post-feeding of 10 days. Measurements were conducted twice a week. The measured values were statistically analyzed for mean value and standard deviation.

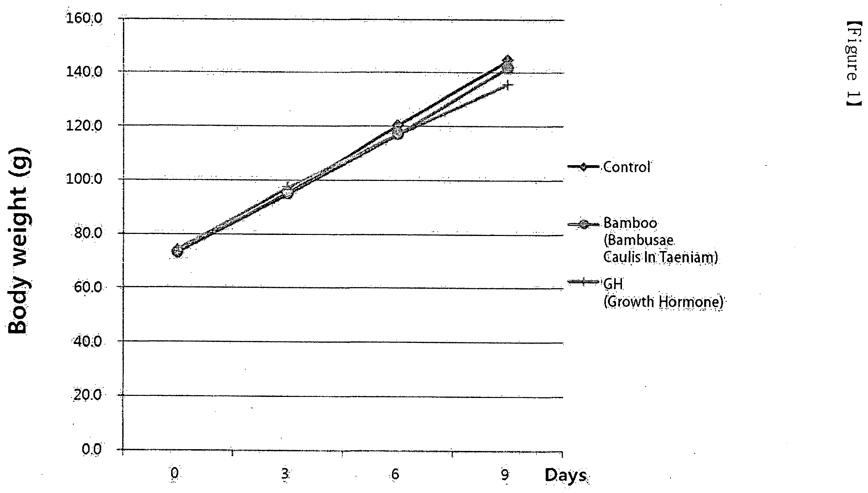

As shown in Figure 1, the observation of 10 days showed no significant difference in weight change among all the groups (i.e. Control group, Bamboo extract-treated group and Growth hormone-treated group). This result implies that the body weight of rats does not significantly correlate with the effect resulting from the administration of the bamboo (*Bambusae caulis in Taeniam*) according to the present invention.

### <1-3> Evaluation on Abnormal Bone Growth and Osteoporosis Toxicity

After 10-day administration as described in Experiment <1-1>, micro-CT scan was conducted to check whether the excessive bone growth or osteoporosis had occurred in the bamboo extract-treated group and the control groups.

The volume and thickness of trabecular bone in the left tibia, which are related to the bone density, were measured by micro-CT scan. After the rats were sacrificed, their left tibia was collected to measure the volume and thickness of its trabecular bone. SKYSCAN 1172 micro-CT scan was employed for micro-CT system, while X-ray source was set at 50kV, 200mA. The source and detector were fixated, while the object rotated between them to produce projected data. Image quality was 0.8 um ∼ 25 um pixel. To calculate the exact ratio, the Micro-CT scanned the overall Field of View (FOV) and the region of interest (ROI) of the left shin splint (See Chunet et al.2004). Subsequently, the FOV region was recomposed to obtain the ratio and thickness of trabeculae. Fuzzy distance transform (FDT) algorithm was applied to compute the volume ratio (BV/TV, %) and thickness (Tb.Th, mm) of trabecular inside the rectangular zone of the left femur as shown in the 2D image (See In Kon Chun 1., et al., In vivo trabecular thickness measurement in cancellous bones: longitudinal rat imaging studies. Physiol. Meas. 27 (2006) 695-702).

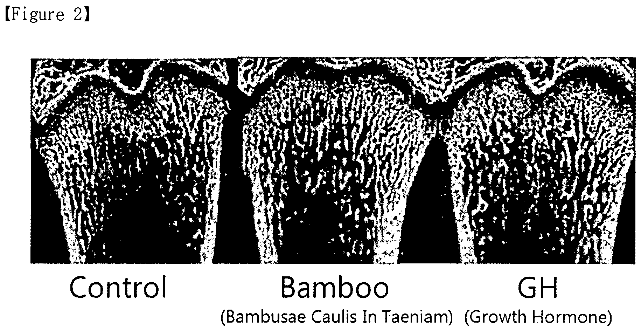

Percentage bone volume, trabecular separation and trabecular thickness as measured by the above micro-CT scan are the indicators for determining the presence of osteoporosis and the abnormality of bone. As seen in Figure 2 and Table 1, there was no significant difference among all the groups, thus demonstrating that abnormal bone proliferation and osteoporosis toxicity had not occurred during the experimental period.

**[Table 1]**

| | Control | *Bamboo*(*Bambusae caulis in Taeniam)* | GH(Growth Hormone) |
|---|---|---|---|
| Percent Bone Volume(%) | 28.66 | 23.26 | 27.87 |
| Bone Surface/Volume ratio(l/mm) | 32.56 | 33.96 | 31.26 |
| Trabecular thickness(mm) | 0.13 | 0.13 | 0.13 |
| Trabecular number(l/mm) | 2.20 | 1.84 | 2.13 |
| Trabecular separation(mm) | 0.32 | 0.39 | 0.34 |

### <1-4> Measurement of Changes in Tibia Length of Experimental Animals

After 10-day administration as described in Experiment <1-1>, both right and left tibias of the rats in the experimental group (i.e. Bamboo (*Bambusae caulis in Taeniam*) extract-treated group) and the control groups were collected, followed by the removal of the muscles, fats and ligaments attached to the bone tissues. Then, the resulting bones were stored in 70% alcohol. Subsequently, X-ray images were obtained by using an X-ray device (OM-FORTE -10121, DK Medical System Co.) with X-ray source set at 50 kV, 200 mA. Tibia length was measured by X-ray radiographs on the tibias.

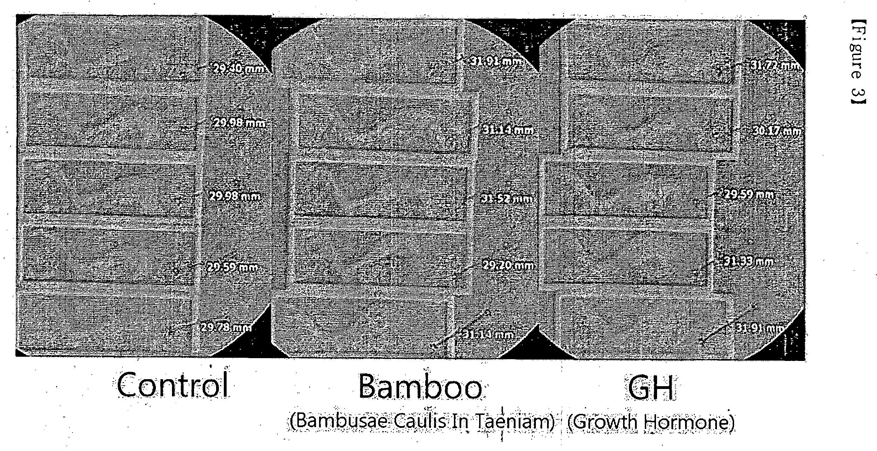

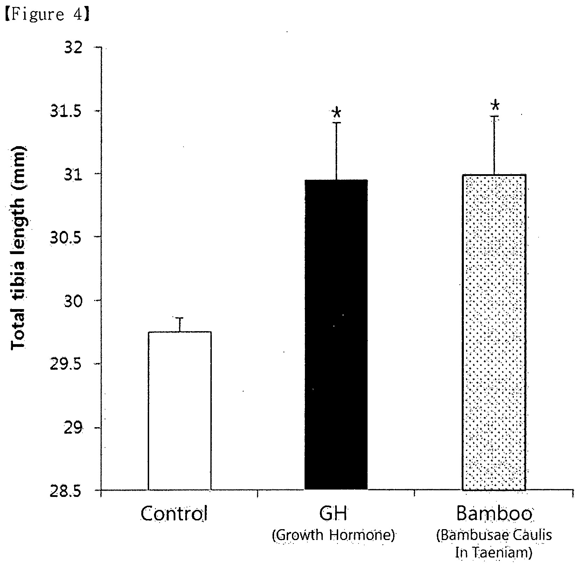

As seen in Figures 3 & 4, the total tibia length in the growth hormone-treated group increased greater than that of the Control group, while the total tibia length in the bamboo (*Bambusae caulis in Taeniam*) extract-treated group also increased greater than the Control group.

### <1-5> Measurement of Changes in Cartilaginous Tissues within the Growth Plate in Experimental Animals

In order to measure the length of the growth plate zone, the cartilaginous tissues were fixated after autopsy, followed by H&E staining and microscope examination. The length of the growth plate zone was measured five(5) times for each sample. The resulting mean values as obtained were used as a representative value for evaluating their significance.

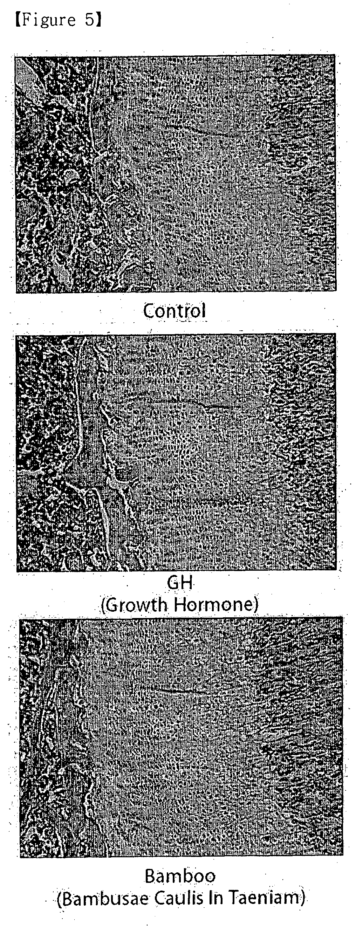

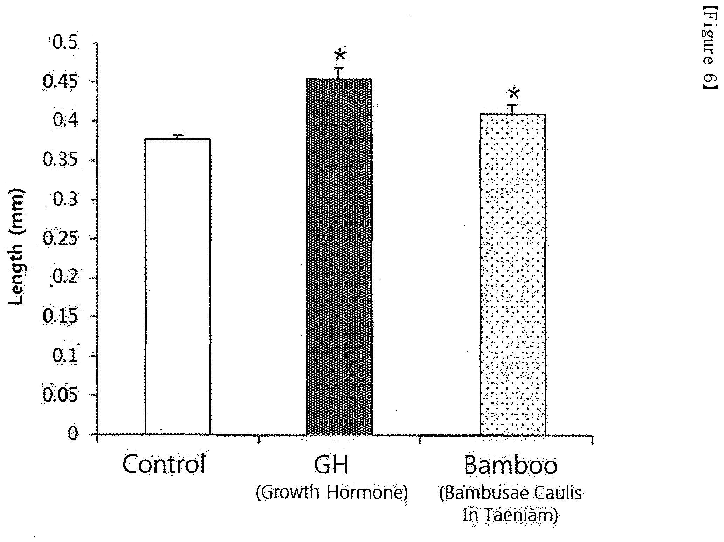

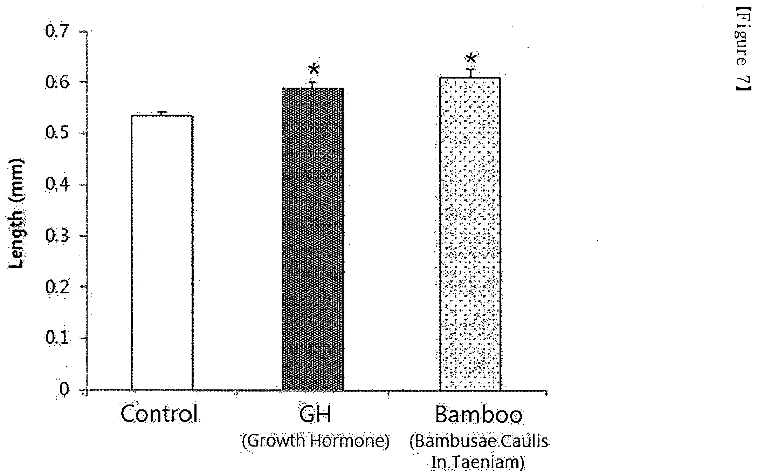

As seen in Figures 5-7, it was shown that the length of the proliferative zone and the hypertrophic zone in the Growth hormone-treated group increased greater than that of the Control group. Likewise, while the length of the proliferative zone of the Bamboo (*Bambusae caulis in Taeniam*) extract-treated group also significantly increased greater than that of the Control group, the length of the hypertrophic zone in the Bamboo (*Bambusae caulis in Taeniam*) extract-treated group increased similar to that of the Growth hormone-treated group.

### <1-6> Proliferative effect of Chondrocytes

In order to measure the extent of chondrocyte proliferation, the cartilaginous tissues of the rat' s tibia were utilized, while BrdU staining technique was applied to proliferative cells at S phase within the growth plates. 30mg/kg of 5-Bromo-2'-deoxyuridine (Product No. B9285, Sigma Co.) was dissolved in 0.1M ammonium hydroxide (NH₄OH). It was then administered to the abdominal cavity of the experimental rats 1 hour prior to the end of the experiment.

After an autopsy was conducted, the cartilaginous tissues were fixated to make paraffin-embedded tissues. Staining was then applied as instructed in BrdU staining kit (Invitrogen, 93-3943). BrdU positive cells were counted by using a cell counter.

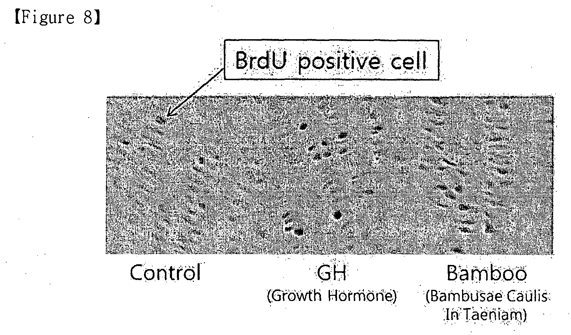

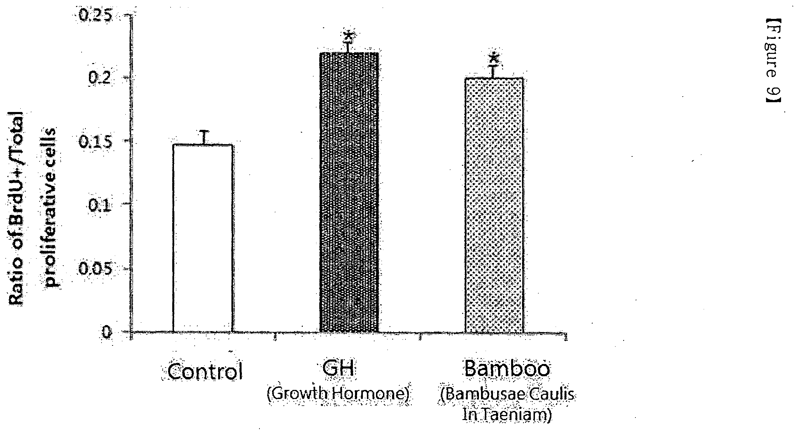

As a result, as seen in Figures 8 & 9, the ratio of BrdU positive cells increased in both GH group (the Growth hormone-treated group) and the Bamboo (*Bambusae caulis in Taeniam*) extract-treated group greater than in the Control group. It demonstrated that the Bamboo (*Bambusae caulis in Taeniam*) extract-treated group is effective for the proliferation of chondrocytes.

### <1-7> Measurement of Osteoblast-activating factor Osteocalcin in the Serum

After the completion of the administration, blood was collected from the hearts of the experimental rats and coagulated. Serum was then separated so as to obtain samples. Subsequently, the total level of osteocalcin in the serum was measured by using Rat Ostecalcin/Bone Gla Protein. OT/BGP ELISA Kit (Cusabio, product no.: CSB-E05129r).

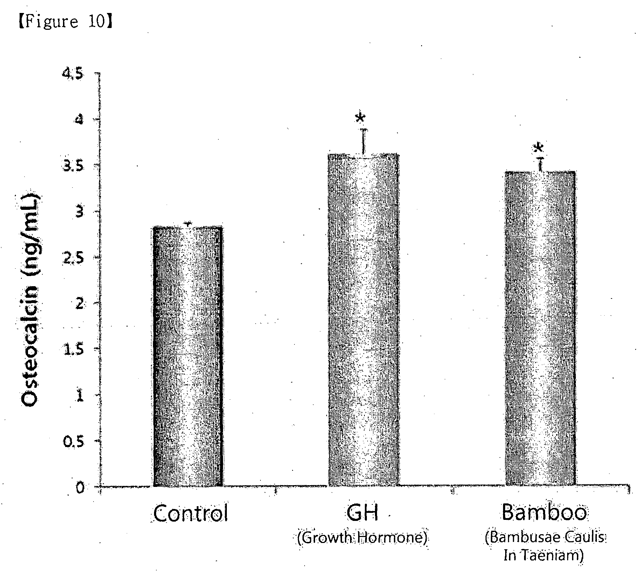

As seen in Figure 10, the level of osteocalcin in the serum significantly increased in both GH group (the Growth hormone-treated group) and the Bamboo (*Bambusae caulis in Taeniam*) extract-treated group, in comparison with the Control group. It is known that osteocalcin is formed in osteoblasts and embedded in the bone matrix, while a certain portion of newly formed osteocalcin is released into the blood stream. Therefore, since measuring the concentration of osteocalcin in the blood allows us to know the extent of osteogenesis, the concentration of osteocalcin in the serum is considered a factor reflecting the activity of osteoblast. As described above, the Bamboo (*Bambusae caulis in Taeniam*) extract according to the present invention was found to significantly increase the concentration of osteocalcin in the serum, implying that it increases osteogenesis and the activity of osteoblast.

### [Industrial Applicability]

As described above, the present invention is directed to a novel use of bamboo for the promotion of bone length growth. More specifically, it is directed to a pharmaceutical composition, a food composition and/or an animal feed composition for promoting bone growth, comprising bamboo as an effective ingredient. Further, it is also directed to a method for promoting bone growth, comprising administering to a subject in need thereof an effective amount of a bamboo.

Since the composition of the present invention comprising bamboo as an effective ingredient is effective for promoting the growth of the growth plates and long bones, it is effective for promoting the growth and the formation of skeletal frame in infants, children and teenagers during their growth period. In addition, the composition of the present invention is effective for the treatment of height growth solely or in combination with growth hormone therapies.

## Claims

1. A non-therapeutic use of a composition for promoting bone length growth, the composition comprising a bamboo extract as an effective ingredient, wherein the bamboo extract comprises Bambusae caulis in Taeniam of Phyllostachys nigra var. henonis, which is the middle parts of bamboo branches whose outer layers are Phyllostachys nigra var. henonis of which external layer is removed.

2. The non-therapeutic use according to claim 1, wherein the bamboo extract is the extract prepared by using at least one solvent selected from the group consisting of water, a C1-C6 alcohol, acetone, ether, chloroform, ethyl acetate, methylene chloride, hexane, cyclohexane, petroleum ether, diethyl ether, benzene, and subcritical or supercritical fluid.

## Patentansprüche

1. Nicht-therapeutische Verwendung einer Zusammensetzung zur Förderung des Knochenwachstums, wobei die Zusammensetzung einen Bambusextrakt als wirksamen Inhaltsstoff umfasst, wobei der Bambusextrakt Bambusae caulis in Taeniam der Phyllostachys nigra var. henonis umfasst, welches die Mittelteile von Bambusästen sind, deren äußere Lagen Phyllostachys nigra var. henonis sind, deren äußere Lage entfernt wurde.

2. Nicht-therapeutische Verwendung nach Anspruch 1, wobei der Bambusextrakt der Extrakt ist, der durch Verwendung zumindest eines der Lösungsmittel gewählt aus der Gruppe bestehend aus Wasser, einem C1-C6 Alkohol, Aceton, Ether, Chloroform, Ethylacetat, Methylenchlorid, Hexan, Cyclohexan, Petrolether, Diethylether, Benzol und unterkritische oder überkritische Flüssigkeit hergestellt ist.

## Revendications

1. Utilisation non thérapeutique d'une composition favorisant la croissance osseuse, dans laquelle la composition comprenant un extrait de bambou comme ingrédient actif, l'extrait de bambou comprenant de l'extrait de bambou dans le Taeniam de Phyllostachys nigra var. henonis, qui sont les parties centrales de branches de bambou dont les couches externes sont Phyllostachys nigra var. henonis, dont la couche externe a été enlevée.

2. Utilisation non thérapeutique selon la revendication 1, dans laquelle l'extrait de bambou est l'extrait obtenu en utilisant au moins l'un des solvants choisis dans le groupe constitué par l'eau, un alcool C1 -C6, l'acétone, l'éther, le chloroforme, l'acétate d'éthyle, le chlorure de méthylène, l'hexane, cyclohexane, éther de pétrole, éther diéthylique, benzène et fluide subcritique ou supercritique.
